(19) Europäisches Patentamt
European Patent Office
Office européen des brevets

(11) Veröffentlichungsnummer : **0 398 154 B1**

(12) # EUROPÄISCHE PATENTSCHRIFT

(45) Veröffentlichungstag der Patentschrift : **23.06.93 Patentblatt 93/25**

(51) Int. Cl.$^5$ : **C07C 31/38,** C07C 29/44

(21) Anmeldenummer : **90108800.5**

(22) Anmeldetag : **10.05.90**

(54) **Verfahren zur Herstellung von Telomeralkoholen.**

(30) Priorität : **13.05.89 DE 3915759**

(43) Veröffentlichungstag der Anmeldung : **22.11.90 Patentblatt 90/47**

(45) Bekanntmachung des Hinweises auf die Patenterteilung : **23.06.93 Patentblatt 93/25**

(84) Benannte Vertragsstaaten : **BE DE FR GB IT NL**

(56) Entgegenhaltungen :
**DE-A- 1 418 313**
**US-A- 2 559 628**
**US-A- 4 346 250**
**INDUSTRIAL AND ENGINEERING CHEMISTRY, internationale Auflage, Band 51, Nr. 7, Juli 1959 Seiten 829-830 The American Chemical Society DONALD R.BAER " Fluoro alcohols"**

(73) Patentinhaber : **HOECHST AKTIENGESELLSCHAFT**
**Postfach 80 03 20**
**W-6230 Frankfurt am Main 80 (DE)**

(72) Erfinder : **Blickle, Peter, Dr.**
**Hattersheimer Strasse 14**
**W-6233 Kelkheim (Taunus) (DE)**
Erfinder : **Heumüller, Rudolf, Dr.**
**Strandpromenade 3**
**W-6054 Rodgau (DE)**
Erfinder : **Hintzer, Klaus, Dr.**
**Rupertusstrasse 3**
**W-8269 Burgkirchen (DE)**
Erfinder : **Schwertfeger, Werner, Dr.**
**Erlenstrasse 8**
**W-6306 Langgöns (DE)**
Erfinder : **Von Werner, Konrad, Dr.**
**Wehrstrasse 6**
**W-8268 Garching (DE)**

**EP 0 398 154 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Telomeralkoholen durch Telomerisation von Tetrafluorethylen mit Methanol bei erhöhtem Druck und erhöhter Temperatur in Gegenwart von radikalbildenden peroxidischen Initiatoren.

Die Umsetzung von Tetrafluorethylen (TFE) mit Methanol zu Telomeralkoholen der allgemeinen Formel

$$H(CF_2\text{-}CF_2)_nCH_2OH,$$

worin n eine ganze Zahl von 1 bis etwa 12 ist, in Gegenwart von peroxidischen Initiatoren, wie beispielsweise Di-tert.-butylperoxid ist bekannt [Industr. Eng. Chem. 51 (1959) Seiten 829 bis 830]. Die Umsetzung erfolgt bei 80 bis 200 °C und bei Drücken von 1 bis etwa 200 bar. Von den von der vorgenannten Formel umfaßten Homologen haben lediglich diejenigen eine industrielle Bedeutung erlangt, in denen n = 1 bis 3 ist. Sie stellen bei Raumtemperatur farblose Flüssigkeiten dar, während die höheren Homologen wachsartige Festkörper sind. Unter den genannten Bedingungen entsteht ein Produktgemisch, in dem das Produkt der obigen Formel mit n = 1 stark überwiegt, während die ebenfalls noch flüssigen Homologen mit n = 2 und 3 in Gegenwart solcher Peroxid-Initiatoren nicht in lohnenswerter Ausbeute erhalten werden können. Gerade diese Homologen mit n = 2 und 3 sind aber als Ausgangsprodukte für die Synthese von höheren omega-H-Perfluoralkylvinylethern (omega-H-Perfluorpentyl- und -heptylvinylether und dessen höhere, um Hexafluorpropylenoxid-Einheiten verlängerte Homologe) von besonderem Interesse, die ihrerseits in Ionenaustauscher-Polymeren Verwendung finden.

In der US-PS 4,346,250 wird ein Verfahren beschrieben, in dem das Verhältnis der Partialdrücke von TFE zu Methanol im Bereich von 30 : 1 bis 1 : 5 gehalten und der Gesamtdruck auf 0,981 bis 11,772 bar (1 bis 12 $kg/cm^2$) Überdruck eingestellt und eine Temperatur von 25 bis 150 °C eingehalten wird. Als Initiatoren werden Di-isopropylperoxydicarbonat, Di-tert.-butylperoxid, tert.-Butylperoxyisobutyrat und Azo-bis-butyronitril genannt. Mit diesem Verfahren ist es zwar möglich, die Bildung von höheren Homologen mit 5 und mehr Tetrafluorethylen-Einheiten weitgehend oder völlig zu unterdrücken. Jedoch besteht auch hier der überwiegende Anteil des Produktes aus den Homologen mit n = 1, die Homologen mit n = 2 und 3 werden nur in untergeordneten Anteilen gebildet. Lediglich beim tert.-Butylperoxyisobutyrat tritt eine Verschiebung zum höheren Anteil des Homologen n = 2 ein, jedoch geht dies einher mit einem Ansteigen des Anteils von n = 4 auf über 5 % des Gesamtproduktes. Es wird außerdem darauf hingewiesen, daß die genannten Initiatoren keine telogene Aktivität aufweisen sollen und daß es ferner dringend geboten ist, ein säurebindendes Mittel zuzusetzen, um dem mit sinkendem pH-Wert im Verlauf der Reaktion eintretenden Abfall der Reaktionsgeschwindigkeit entgegenzuwirken. Die erstere Forderung ist besonders schwierig zu erfüllen, da diese Peroxide häufig handelsüblich als Suspension oder Lösung in sogenannten Phlegmatisierungsmitteln geliefert werden. Dies sind meist aliphatische Kohlenwasserstoffe mit einem Kochpunkt von 170 bis 300 °C, wie beispielsweise Benzin, Decan, Isodecan oder Isododecan, die eine beträchtliche telogene Aktivität aufweisen. Somit ist eine möglichst sorgfältige Entfernung dieses Mittels erforderlich, da dieses sonst in unerwünschter Weise in den Reaktionsablauf eingreift und mit den TFE- und Methanol-Radikalen reagiert.

Es bestand somit die Aufgabe, die vorgenannten Nachteile zu vermeiden. Dies wird erfindungsgemäß erreicht durch ein Verfahren der eingangs genannten Art, das dadurch gekennzeichnet ist, daß die Telomerisation unter einem Tetrafluorethylendruck von 2 bis 10 bar und bei einer Temperatur von 20 bis 70 °C in Gegenwart von Perestern der allgemeinen Formel

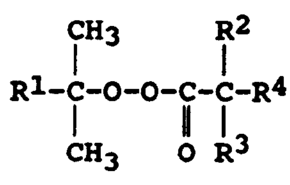
,

worin $R^1 = CH_3$, $C_2H_5$ oder $C_6H_5$ ist und $R^2$, $R^3$ und $R^4$ Alkylreste von 1 bis 6 C-Atomen darstellen, unter Anlagerung von vorwiegend 1 bis 3 Tetrafluorethylen-Einheiten geführt wird.

Solche Initiatoren sind beispielsweise tert.-Amylperoxypivalat, tert.-Butylperoxyneodecanoat und Cumylperoxyneodecanoat. Besonders bevorzugt ist es, die Telomerisation in Gegenwart von tert.-Butylperoxypivalat durchzuführen. Dabei werden die genannten Initiatoren in einer Gesamtmenge von 0,1 bis 20, vorzugsweise von 0,3 bis 10 Mol-%, bezogen auf TFE, eingesetzt. Die Gesamtmenge an Initiator kann am Anfang der Reaktion vorgelegt oder zugegeben werden. Man kann jedoch auch nur eine Teilmenge des Initiators am Anfang hinzufügen und weitere Teilmengen im Verlauf der Reaktion kontinuierlich oder stufenweise nachdosieren, bis die Reaktion einen Umsetzungsgrad von 80 % erreicht hat. Dabei wird eine Reaktionstemperatur von 20 bis 70 °C, vorzugsweise von 35 bis 70 °C, eingehalten und ein Druck von 2 bis 10 bar, vorzugsweise von 3 bis 8 bar, durch Aufpressen und Nachführen von TFE eingestellt und gehalten.

Durch entsprechende Wahl von Druck und Temperatur kann die Reaktion sowohl auf den Telomeralkohol mit n = 1 in der obigen Formel als auch auf denjenigen mit n = 2 als Hauptprodukt gesteuert werden. Dies zeigt die Tabelle im untenstehenden Beispiel 1. Dabei treten die höheren Telomeralkohole, die wegen ihrer wachsartigen Konsistenz die Aufarbeitung stören, selbst bei sehr niedrigen Reaktionstemperaturen nur in ganz untergeordnetem Maße auf. Eine Säurebildung während der Reaktion und deren nachteilige Folgen werden nicht beobachtet, so daß ein besonderer Zusatz säurebindender Mittel entfällt.

Das erfindungsgemäße Verfahren wird etwa wie folgt durchgeführt: Flüssiges Methanol wird in der erforderlichen Menge vorgelegt und auf die gewünschte Reaktionstemperatur erwärmt. Sodann wird der Initiator zugegeben, entweder in seiner Gesamtmenge oder in einer Teilmenge, wobei der Rest kontinuierlich oder stufenweise nachdosiert wird. Vorzugsweise wird der Initiator bei größeren Reaktionsansätzen nachdosiert. Die Initiatorzugabe erfolgt als 50- bis 80gew.%ige Lösung in einem aliphatischen Kohlenwasserstoff, meist in Isododecan. Sodann wird TFE bis zum gewünschten Druck aufgepreßt und unter Einhaltung dieses Druckes nachgeführt. Die Reaktion erfolgt unter Rühren so lange, bis die TFE-Aufnahme stark abfällt. Anschließend wird das Produkt destilliert, und zwar zunächst zur Abtrennung des überschüssigen Methanols. Danach werden die Telomeralkohol-Fraktionen durch fraktionierte Destillation getrennt, wobei als eine Fraktion das Phlegmatisierungsmittel anfällt.

Die genannten Telomeralkohole der oben bezeichneten Formel mit n = 1 bis 3 finden vor allem Verwendung für die Herstellung von deren Acrylatestern, die Zwischenprodukte für Textilhilfsmittel sind. Diese Telomeralkohole sind außerdem Ausgangsprodukte für die Herstellung von omega-H-Perfluorpropylvinylether und dessen höheren Homologen, die ihrerseits wichtige Ausgangsprodukte für die Herstellung wertvoller Polymerer sind.

Die Erfindung wird durch folgende Beispiele erläutert.

Beispiele 1a bis 1i

Ein mit Stickstoff gespülter Kessel aus V4A-Stahl, ausgerüstet mit einem Impellerrührer, wird mit 15 kg an sauerstofffreiem Methanol beschickt und anschließend noch zweimal mit TFE gespült. Der Kesselinhalt wird danach auf die jeweils gewünschte Reaktionstemperatur (siehe Tabelle) gebracht. Nun werden in den Kessel 300 g einer 75gew.%igen Lösung von tert.-Butylperoxypivalat (= 1,29 mol) in Isododecan hinzugegeben und dann TFE bis zum entsprechenden Druck (siehe Tabelle) aufgepreßt. TFE wird kontinuierlich unter konstant gehaltenem Druck nachgeführt.

Nachdem der Ansatz kein TFE mehr aufnimmt, wird der Kesselinhalt abgekühlt, entspannt und das Methanol wird abdestilliert. Die Zusammensetzung des Telomeralkoholgemisches wird durch Gaschromatographie (GC) bestimmt und ist in der folgenden Tabelle angegeben.

TABELLE

| Beispiele | Reaktions-temperatur (°C) | TFE-Druck (bar) | TFE-Aufnahme (mol) | Anteile der Fraktionen (Gew.-%) *) n = 1 | n = 2 | n = 3 | n = 4 | n = 5 |
|---|---|---|---|---|---|---|---|---|
| 1a | 70 | 2 | 110 | 78,6 | 19,8 | 1,6 | --- | --- |
| 1b | 70 | 3 | 115 | 70,2 | 27,5 | 2,3 | --- | --- |
| 1c | 65 | 2 | 110 | 69,7 | 27,0 | 3,2 | 0,1 | --- |
| 1d | 65 | 4 | 180 | 55,4 | 39,1 | 4,9 | 0,6 | --- |
| 1e | 65 | 6 | 180 | 50,1 | 41,5 | 7,0 | 1,3 | 0,1 |
| 1f | 65 | 10 | 210 | 42,9 | 42,7 | 12,6 | 1,6 | 0,2 |
| 1g | 60 | 10 | 220 | 36,3 | 43,7 | 17,1 | 2,5 | 0,4 |
| 1h | 55 | 10 | 220 | 31,6 | 45,7 | 20,4 | 1,8 | 0,5 |
| 1i | 50 | 10 | 220 | 22,9 | 49,1 | 24,3 | 3,1 | 0,6 |

*) n bezieht sich auf die oben angegebene allgemeine Formel der Telomeralkohole

Beispiel 2

Gemäß der Vorschrift von Beispiel 1 werden Methanol und TFE bei einem Druck von 5 bar bei 65 °C, aber

unter Zugabe von 500 g einer 75gew.%igen Lösung von tert.-Amylperoxypivalat (1,99 mol), umgesetzt. Folgende durch GC bestimmte Fraktionen wurden erhalten:

n = 1 35,7 Gew.-%
n = 2 40,6 Gew.-%
n = 3 18,9 Gew.-%
n = 4 4,1 Gew.-%
n = 5 0,7 Gew.-%

## Patentansprüche

1. Verfahren zur Herstellung von Telomeralkoholen durch Telomerisation von Tetrafluorethylen mit Methanol bei erhöhtem Druck und erhöhter Temperatur in Gegenwart von radikalbildenden peroxidischen Initiatoren, dadurch gekennzeichnet, daß die Telomerisation unter einem Tetrafluorethylendruck von 2 bis 10 bar und bei einer Temperatur von 20 bis 70 °C in Gegenwart von Perestern der allgemeinen Formel

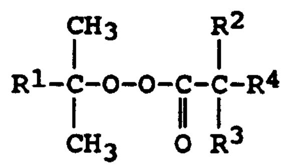,

worin $R^1$ = $CH_3$, $C_2H_5$ oder $C_6H_5$ ist und $R^2$, $R^3$ und $R^4$ Alkylreste von 1 bis 6 C-Atomen darstellen, unter Anlagerung von vorwiegend 1 bis 3 Tetrafluorethylen-Einheiten geführt wird.

2. Verfahren zur Herstellung von Telomeralkoholen gemäß Anspruch 1, dadurch gekennzeichnet, daß die Telomerisation in Gegenwart von tert.-Butylperoxypivalat geführt wird.

## Claims

1. A process for the preparation of telomer alcohols by telomerisation of tetrafluoroethylene with methanol at elevated pressure and elevated temperature in the presence of free radical-forming peroxide initiators, characterized by carrying out the telomerisation under a tetrafluoroethylene pressure of 2 to 10 bars and at a temperature of 20 to 70 °C in the presence of peresters of the formula

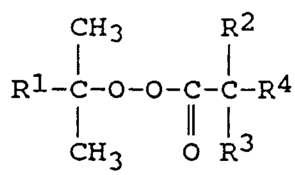

in which $R^1$ is $CH_3$, $C_2H_5$ or $C_6H_5$ and $R^2$, $R^3$ and $R^4$ represent alkyl radicals having 1 to 6 carbon atoms, adding on in the main 1 to 3 tetrafluoroethylene units.

2. The process for the preparation of telomer alcohols according to claim 1, characterized in that the telomerisation is carried out in the presence of tert.-butyl peroxypivalate.

## Revendications

1. Procédé pour préparer des alcools télomères par télomérisation du tétrafluoréthylène avec du méthanol sous pression élevée à température élevée, en présence d'amorceurs peroxydiques générateurs de radicaux libres, procédé caractérisé en ce qu'on conduit la télomérisation sous une pression de tétrafluoréthylène de 2 à 10 bars et à une température de 20 à 70°C, en présence de peresters de formule générale :

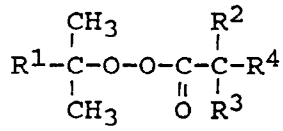

(dans laquelle $R^1$ représente $CH_3$, $C_2H_5$ ou $C_6H_5$; et $R^2$, $R^3$ et $R^4$ représentent des restes alkyles ayant 1 à 6 atomes de carbone), avec, de façon prédominante, fixation de 1 à 3 motifs tétrafluoréthylènes.

**2.** Procédé de préparation de télomères d'alcools selon la revendication 1, caractérisé en ce qu'on effectue la télomérisation en présence de peroxypivalate de tert.-butyle.